Europäisches Patentamt

European Patent Office

Office européen des brevets

(19)

(11) Veröffentlichungsnummer: **0 357 028**
**A2**

(12) **EUROPÄISCHE PATENTANMELDUNG**

(21) Anmeldenummer: **89116000.4**

(22) Anmeldetag: **30.08.89**

(51) Int. Cl.5: **C07H 21/04 , C12Q 1/68 , C12N 15/01**

Der Anmelder hat eine Erklärung nach Regel 28 (4) EPÜ (Herausgabe einer Probe nur an einen Sachverständigen) eingereicht.
Eingangsnummer(n) der Hinterlegung(en): DSM 4772.

(30) Priorität: **31.08.88 DE 3829535**

(43) Veröffentlichungstag der Anmeldung:
**07.03.90 Patentblatt 90/10**

(84) Benannte Vertragsstaaten:
**AT BE CH DE ES FR GB GR IT LI LU NL SE**

(71) Anmelder: **Biotest AG**
**Flughafenstrasse 4**
**D-6000 Frankfurt am Main 73(DE)**

(72) Erfinder: **Holtkamp, Bodo, Dr.**
**Mühlstrasse 62**
**D-6054 Rodgau 1(DE)**
Erfinder: **Bohnke, Eberhard**
**Würzburger Strasse 36**
**D-6000 Frankfurt am Main 60(DE)**
Erfinder: **Sonneborn, Hans-Hermann, Dr.**
**Im Birkeneck 70**
**D-6056 Heusenstamm(DE)**
Erfinder: **Rittner, Christian, Prof. Dr.**
**Höhenweg 8**
**D-8501 Nieder-Olm(DE)**
Erfinder: **Schneider, Peter Mathias, Dr.**
**Friedrich-Ebert-Strasse 33**
**D-6501 Budenheim(DE)**
Erfinder: **Schacker, Ulrike**
**Watfordstrasse 18**
**D-6500 Mainz(DE)**

(74) Vertreter: **Vossius & Partner**
**Siebertstrasse 4 P.O. Box 86 07 67**
**D-8000 München 86(DE)**

(54) **Nukleinsäure-Fragmente aus eukaryontischen Genomen, Verfahren zu ihrer Herstellung und ihre Verwendung zum Nachweis von Restriktions-Fragment-Längen-Polymorphismen (RFLPs).**

(57) Beschrieben werden Nukleinsäure-Fragmente aus eukaryontischen Genomen, die zum Nachweis von Restriktions-Fragment-Längen-Polymorphismen (RFLPs) in eukaryontischen Genomen geeignet sind. Sie sind erhältlich durch Absuchen einer Genbank eines Eukaryonten mit dem NciI/ClaI-Fragment aus dem Protein III-Gen des Bakteriophagen M13. Sie verursachen die Bindung von mindestens fünfmal mehr rekombinanten DNA-Molekülen, in denen sie enthalten sind, an eine zu ihnen homologe Zielsequenz als eine Vektor-DNA an eine zu ihr homologe Zielsequenz.
Ferner werden Verfahren zur Isolierung dieser Nukleinsäure-Fragmente beschrieben.

## Nukleinsäure-Fragmente aus eukaryontischen Genomen, Verfahren zu ihrer Herstellung und ihre Verwendung zum Nachweis von Restriktions-Fragment-Längen-Polymorphismen (RFLPs)

Die Erfindung betrifft Nukleinsäure- Fragmente aus eukaryontischen Genomen, Verfahren zu ihrer Isolierung und Synthese, sowie ihre Verwendung zum Nachweis von Restriktions-Fragment-Längen-Polymorphismen (RFLP's) bei Eukaryonten.

Polymorphe Sequenzen in Eukaryonten-Genomen können für die Indentifizierung von Individuen und für die Ermittlung von Verwandtschaftsbeziehungen zwischen Individuen benutzt werden. Eine Möglichkeit zum Nachweis von Sequenzpolymorphismen beruht auf der Sequenz-spezifischen Spaltung genomischer DNA durch Restriktionsenzyme: Wird nämlich genomische DNA einer Restriktionsspaltung durch ein Enzym unterworfen, welches den DNA-Doppelstrang an definierten Sequenzen (Restriktionsspaltstellen) spaltet, so entstehen abhängig von der Position der vom Restriktionsenzym erkannten Sequenzen unterschiedlich lange Fragmente. Die Genome aller somatischen Zellen eines Individuums sind abgesehen von seltenen somatischen Mutationen identisch. Deshalb erhält man nach einer vollständigen Restriktionsspaltung, unabhängig von dem Gewebe, aus dem die DNA isoliert wurde, die gleichen Restriktionsfragmente. Genetisch verschiedene Individuen zeigen jedoch Sequenzunterschiede, durch die unter anderem die Spaltstellen eines Restriktionsenzyms oder die Länge der zwischen zwei Spaltstellen liegenden Sequenz verändert sein können. Die sich daraus ergebende Variabilität in der Länge von Restriktionsfragmenten an einem genetischen Locus bezeichnet man als Restriktions-Fragment-Längen-Polymorphismus (RFLP). Da Veränderungen der DNA Sequenz durch Mutationen auch in der Keimbahn selten sind, werden Restriktionsspaltstellen stabil vererbt. Deshalb ergibt sich eine 50-prozentige Identi.ät zwischen den Restriktionsfragmenten eines Elterns mit jedem seiner Nachkommen. Die Restriktionsfragmente lassen sich deshalb als Merkmale sowohl für die Individuentypisierung als auch für den Nachweis von Verwandtschaftsverhältnissen nutzen. Für die Analyse von RFLPs wird die Methode des Southern Blot (Southern, E., J. Mol. Biol, 98, 503-517, 1975) eingesetzt. Dabei werden die nach einem Restriktionsverdau entstandenen doppelsträngigen DNA-Fragmente zunächst elektrophoretisch entsprechend ihrer Größe getrennt, in Einzelstränge zerlegt und an einen festen Träger gebunden. Der Nachweis der gesuchten Fragmente erfolgt mit einer markierten Nukleinsäure-Sonde, die mit einzelsträngigen Restriktionsfragmenten, welche Sequenzhomologie zur Sonde aufweisen, über Basenpaarbindungen zum Doppelstrang hybridisiert. Die Position jedes Restriktionsfragmentes, welches mit der Sonde hybridisiert, ist aufgrund der Markierung der Sonde als Bande nachweisbar.

Die Menge an genetischer Information, die mit einer Sonde erhalten werden kann, ist abhängig von der Zahl der genetischen Loci mit Sequenzhomologie zur Sonde und vom Grad des Polymorphismus in den Loci. Es sind in der Literatur eine Reihe von Sonden mit Homologie zu hochpolymorphen, repetitiven Sequenzen (Minisatelliten) beschrieben (Jeffreys, A.J., et al., Nature 314, 67-73, 1985; EP-A1 0 186 271; Vassart, G., et al., Science 235, 683-684, 1987; EP-A2 0 264 305; Georges, M., et al., Nucl. Acids Res. 15, 7193, 1987). Bei Minisatelliten wird die Länge der Restriktions-Fragmente wesentlich durch die Zahl der Widerholungen des Grundmotivs der repetitiven Sequenzen bestimmt, vorausgesetzt, die vom Restriktionsenzym erkannte Sequenz ist nicht Teil der repetitiven Sequenz. Da Mini satelliten-Sonden eine Homologie zu einer Reihe von genetischen Loci im Eukaryonten-Genom aufweisen, hybridisieren sie mit einer Vielzahl von Restriktionsfragmenten und liefern ein für ein Individuum spezifisches Bandenmuster, d.h. einen "genetischen Fingerabdruck" mit einem hohen Informationswert für die genetische Typisierung von Individuen und für die Feststellung von Verwandtschaftsbeziehungen.

Die Banden eines "Fingerabdrucks" sind von unterschiedlicher Intensität. Die Intensität einer Bande wird wesentlich durch die Zahl der gebundenen Sondenmoleküle bestimmt. Die Bindung ist abhängig von der Stärke der Homologie zwischen Sonde und Ziel-Sequenz, der Länge der homologen Sequenz und den Hybridisierungsbedingungen (Salzkonzentration, Temperatur). Um auch Banden geringer Intensität zu erfassen, ist der Einsatz von Nachweissystemen mit extrem hoher Empfindlichkeit erforderlich. Deshalb werden bislang die Sonden für "genetische Fingerabdrücke" radioaktiv markiert. Durch die Notwendigkeit der radioaktiven Markierung ist die Zahl der Anwender von Minisatelliten-Sonden begrenzt. Um die Sonden einem breiteren Anwenderkreis zugänglich zu machen, ist die Einführung von nicht-radioaktiven Nachweistechniken erforderlich.

Bei den meisten nicht-radioaktiven Methoden werden Sonden verwendet, die mit Biotin markiert sind. Der Nachweis der gebundenen Sonde erfolgt durch eine enzymatische Reaktion, wobei das Enzym nach der Hybridisierung über Biotin-Avidin- (bzw. Biotin-Streptavidin-) Brücken an die Sonde gebunden wird (Leary, J.J., et al., Proc. Natl. Acad. Sci. USA 80, 4045-4049, 1983; Forster, A.C., et al.,

Nucl. Acids Res. 13, 745-761, 1985; Viscidi, R.P., et al., J. Clin. Microb. 23, 311-317, 1986). Es konnte gezeigt werden, daß die Empfindlichkeit von enzymatischen Nachweissystemen in Verbindung mit Biotin-markierten Sonden ausreicht, um Sequenzen, die nur einmal im haploiden Eukaryonten-Genom vorkommen ("Single Copy" Gene), nachzuweisen (Dykes, D., et al., Elektrophoresis 7, 278-282, 1986; Sheldon, E.L., et al., Proc. Natl. Acad. Sci. USA 83, 9085-9089, 1986; McInnes et al., Bio/Technology 5, 269-272, 1987).

Da, wie oben erwähnt, einige der Banden in "genetischen Fingerabdrücken" nur eine geringe Intensität aufweisen, würde der Einsatz von nicht-radioaktiven Nachweistechniken durch eine Signalverstärkung erleichtert. Eine Möglichkeit, eine solche Verstärkung zuerreichen, liegt in der Erhöhung der Zahl der pro Zielmolekül gebundenen Sondenmoleküle. Vorschläge dazu sind in EP-A1 0 124 221, in EP-A3 0 204 510 und in WO 87/03622 beschrieben. Die Signalverstärkung wird dabei durch die Bildung eines Komplexes aus mindestens zwei Typen von Sonden erreicht. Die Sonden des ersten Typs bestehen aus zwei Teilen mit unterschiedlicher Funktion. Der erste Teil weist Homologie zur gesuchten Zielsequenz auf, der zweite Teil besitzt Homologie zu mindestens einer Sonde des zweiten Typs. Die Sequenzen der Sonden sind so gewählt, daß an jedes Sondenmolekül vom Typ 1 mehrere Sondenmoleküle vom Typ 2 binden können, wodurch die Zahl der pro Zielmolekül gebundenen Sondenmoleküle erhöht wird. Bei diesem Verfahren sind entweder mehrere aufeinanderfolgende Hybridisierungsschritte oder eine Hybridisierung mit Gemischen von Sondenmolekülen erforderlich. Somit liegt der Erfindung das technische Problem zugrunde, ein Nukleinsäure-Fragment bereitzustellen, das zur Erkennung von RFLPs von Eukaryonten mit hoher Nachweisempfindlichkeit geeignet ist und diesem Zweck auch ohne radioaktive Markierung verwendet werden kann.

Ferner liegt der Erfindung das technische Problem zugrunde, ein Verfahren zur Isolierung oder Synthese der Nukleinsäure-Fragmente mit den genannten Eigenschaften bereitzustellen.

Die Lösung des genannten technischen Problems wird durch die Bereitstellung der in den Patentansprüchen gekennzeichneten Gegenstände erzielt.

Erfindungsgemäß werden somit Nukleinsäure-Fragmente aus eukaryontischen Genomen bereitgestellt, die die folgenden Merkmale aufweisen;

(a) sie sind erhältlich durch Absuchen einer Genbank eines Eukaryonten mit dem NciI/ClaI-Fragment aus dem Protein III-Gen des Bakteriophagen M13; und

(b) sie verursachen die Bindung von mindestens fünf- bis zehnmal mehr rekombinanten DNA-Molekülen, in denen sie enthalten sind, an eine zu ihnen homologe Zielsequenz als eine Vektor-DNA an eine zu ihr homologe Zielsequenz.

Vorzugsweise enthalten die erfindungsgemäßen Nukleinsäure-Fragmente eine mindestens einmal wiederholte Sequenz mit der Basenreihenfolge 5'- CGGCGGWGGTGGTGGTATYGGTTGTGS - 3' in der W entweder T oder A, S entweder G oder C, und Y entweder C oder T bedeutet.

Die erfindungsgemäßen Nukleinsäure-Fragmente sind DNA- oder RNA-Fragmente, vorzugsweise DNA-Fragmente.

Die erfindungsgemäßen Nukleinsäure-Fragmente sind insbesondere als Sondenmoleküle zum Nachweis von Minisatelliten-Sequenzen von Eukaryonten und von darin vorkommenden RFLPs geeignet.

Sie unterscheiden sich von den in EP-A1 0 186 271 und in EP-A2 0 264 305 beschriebenen Nukleinsäure-Fragmenten durch ihre Sequenz, den "genetischen Fingerabdruck", und den bei ihrer Verwendung auftretenden Verstärkungseffekt. Dieser beruht auf einem strukturellen Unterschied der erfindungsgemäßen Nukleinsäure-Fragmente von den in EP-A1 0 186 271 und EP-A2 0 264 305 beschriebenen Nukleinsäure-Fragmente.

Gemäß EP-A2 0 264 305 werden vollständige M13-Moleküle zum Nachweis von RFLPs bei Menschen und Tieren verwendet. Dabei tritt kein Verstärkungseffekt auf. Erfindungsgemäß werden dagegen mit M13-Molekülen hybridisierende Nukleinsäure-Fragmente aus eukaryontischen Genomen verwendet, die strukturell unterschiedlich vom M13-Molekül sein müssen, da bei ihrer Verwendung ein Verstärkungseffekt auftritt.

Der Vorteil gegenüber den bekannten eingangs erläuterten Signalverstärkungsschemata besteht darin, daß die Erkennung der Zielsequenz und die Signalverstärkung durch Sequenz-identische Nukleinsäure-Fragmente, also durch Nukleinsäure-Fragmente eines Typs erzielt wird, da die Zielsequenz selbst zur Signalverstärkung beiträgt.

In einer bevorzugten Ausführungsform stammen die Nukleinsäure-Fragmente aus dem Genom von Säugern.

In einer besonders bevorzugten Ausführungsform stammen die Nukleinsäure-Fragmente aus dem Genom des Menschen.

Eine der besonders bevorzugten Ausführungsformen ist das im Plasmid pUC-MZ1.3 enthaltene 1.9 kb PstI/Bam HI-Restriktionsfragment aus einem menschlichen Genom. Die DNA-Sequenz dieser Insertion läßt sich in an sich bekannter Weise bestimmen.

Das Plasmid pUC-MZ1.3 wurde am 23.08.88 bei der Deutschen Sammlung von Mikroorganismen (DSM), Braunschweig, Bundesrepublik Deutschland, nach den Bestimmungen des Budapester Vertrages unter der Hinterlegungsnummer DSM 4772 hinterlegt.

Im Plasmid pUC-MZ1.3 ist die vorstehend spezifizierte DNA-Sequenz enthalten.

Die erfindungsgemäßen Nukleinsäure-Fragmente können einzel- oder doppelsträngig vorliegen. Außerdem können sie aus partiell oder vollständig homologen Einzelsträngen zusammengesetzt sein.

Die erfindungsgemäßen Nukleinsäure-Fragmente können radioaktiv markiert werden. Aufgrund des bei ihrer Verwendung auftretenden Verstärkungseffektes sind aber auch erfindungsgemäße Nukleinsäure-Fragmente, die mit Fluoreszenzmarkierungen, enzymatischen Markierungen oder mit chemischen Gruppen versehen sind, die als molekulare Brücke zu einer anderweitigen Markierung dienen, zum Nachweis von RFLPs von Eukaryonten geeignet.

Die erfindungsgemäßen Nukleinsäure-Fragmente lassen sich in einer eukaryontischen Genbank durch Hybridisierung mit einem vollständigen M13-Genom identifizieren, vorzugsweise mit dem NciI/ClaI-Fragment aus dem Portein III-Gen des M13-Genoms. Sodann können die identifizierten Sequenzen in an sich bekannter Weise aus der Genbank isoliert werden. Die bei diesem Verfahren anwendbaren Hybridisierungsbedingungen sind 40 - 45°C, 0.4 - 0.9 M NaCl, 40 -50 % Formamid, pH 7 - 7.5, vorzugsweise 42°C, 0.9 M NaCl, 40 % Formamid, pH 7.4. Die Stringenzwaschschritte werden bei 65°C bis 0.3 - 0.03 M NaCl, pH 7 - 7.5 vorzugsweise bis 0.15 M NaCl, pH 7.4 durchgeführt. Es handelt sich dabei also um übliche Hybridisierungsbedingungen.

In einer anderen Ausführungsform des geschilderten Isolierungsverfahrens kann die Identifizierung erfindungsgemäßer Nukleinsäure-Fragmente durch Hybridisierung mit Nukleinsäure-Fragmenten erfolgen, die selbst wie vorstehend beschrieben isoliert wurden.

Schließlich können erfindungsgemäße Nukleinsäure-Fragmente durch DNA-Synthese hergestellt werden, die unter Berücksichtigung einer DNA-Sequenz eines wie vorstehend isolierten Nukleinsäure-Fragments erfolgt. Bei der Synthese können auch mehrere DNA-Sequenzen berücksichtigt und eine diesen Sequenzen entsprechende Konsensussequenz synthetisiert werden.

Die Figuren zeigen:

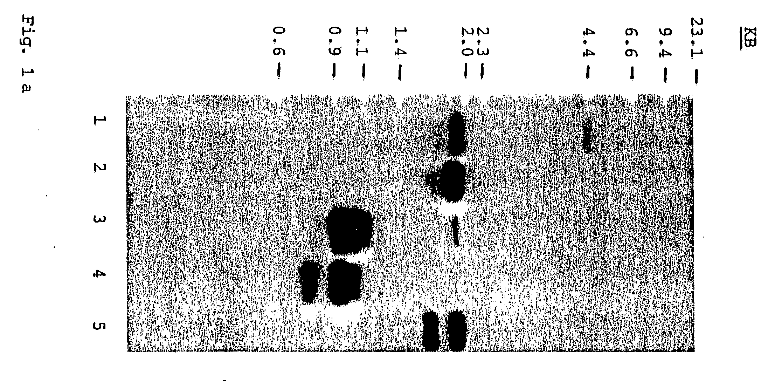

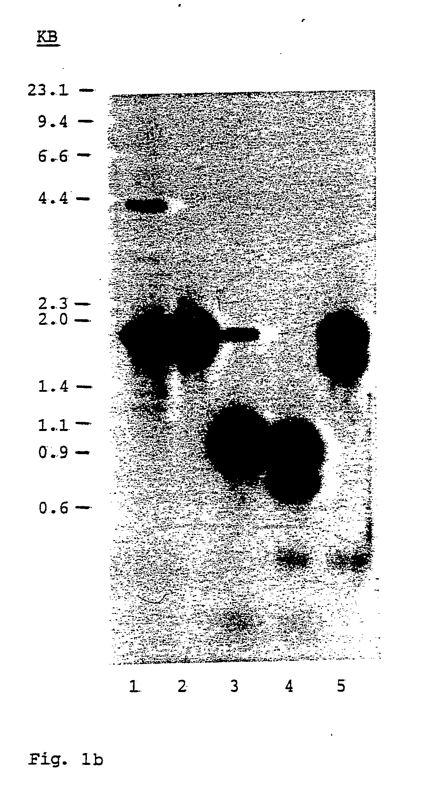

Figur 1: Analyse der menschlichen Insertion des Bakteriophagen-Klones MZ1.3 durch Restriktionskartierung im Southern Blot.
a. 2 Std. Belichtungszeit
b. 16 Std. Belichtungszeit
0,8% Agarosegel; Spur 1: 1μg vollständig PstI/BamHI gespaltene DNA aus Lambda MZ1.3; Spuren 2 - 5: 100 ng gereinigtes 1,9 KB PstI/BamHI Fragment aus MZ1.3; Spur 2: ganzes Fragment; Spur 3: partiell MspI gespalten; Spur 4: vollständig Msp I und partiell Sau 3A gespalten; Spur 5: partiell Sau 3A gespalten. Sonde: 282 bp Hae III/Cla I-Fragment aus dem Protein III-Gen von M13, spez. Aktivität 1,4 x $10^8$ cpm/μg, 3 x $10^5$ cpm/ml Hybr. Lsg.; Hybridisierungs-Puffer: 5 x SSC, 1% SDS, 10 mM Tris/HCl (pH 8,0), 2mM EDTA, 5 μg/ml t-RNA, 2% Trockenmilchpulver. Vorhybridisierung: 60°C, 3 Std. Hybridisierung: 60°C, 20 Std., Waschschritte: 2 x 15 min. in 2 x SSC, 0,1 % SDS bei Raumtemperatur, 2 x 30 min. in 2 x SSC, 0,1 % SDS bei 65°C. Belichtung von Kodak XAR-5 Röntgenfilm, 1 Std. bei Raumtemperatur.

Figur 2: Restriktionskarte des 1,9 KB MZ1.3-Fragmentes in pUC18.
Restriktionsschnittstellen: PstI ↑, BamHI ●, MspI ▽, Sau 3A x (MspI und Sau 3A Schnittstellen im Vektor sind nicht angegeben).

Figur 3: Indentifizierung von Restriktionsfragmenten des M13 Genoms mit Homologie zur Insertion von pUC-MZ1.3.
0,6 % Agarosegel, pro Spur wurden 100 ng durch Restriktionsenzyme gespaltene M13 DNA aufgetragen. Spuren 1 und 4: ClaI Spaltung; Spuren 2 und 5: ClaI/NciI-Spaltung; Spuren 3 und 6: NciI-Spaltung. Sonden: Spuren 1 - 3: M13, spez. Aktivität: 5,4 x $10^8$ cpm/μg, 1 x $10^6$ cpm/ml Hybr. Lsg., Spuren 4 - 6: MZ1.3 (1,9 kb PstI/BamHI Fragment), spez. Aktivität: 1,7 x $10^9$ cpm/μg, 1 x $10^6$ cpm/ml Hybr. Lsg. Hybridisierungs-Puffer: 5 x SSC, 1 % SDS, 10 mM Tris/HCl (pH 8,0), 2 mM EDTA, 5 μg/ml t-RNA, 2% Trockenmilchpulver. Vorhybridisierung: 60°C, 3 Std.; Hybridisierung: 60°C, 20 Std.; Waschschritte: 2 x 15 min. in 2 x SSC, 0,1 % SDS bei Raumtemperatur; 2 x 30 min. in 2 x SSC, 0,1% SDS bei 65°C. Belichtung von Kodak XAR-5 Röntgenfilm, 1 Std. bei Raumtemperatur.

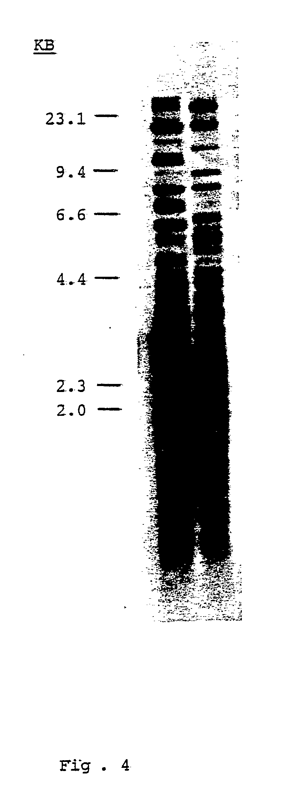

Figur 4: Analyse von RFLP-Fragmenten zweier nicht verwandter Blutspender mit der radioaktiv markierten Sonde pUC-MZ1.3.
Restriktionsspaltung: 10 μg DNA, 40 U Hinfl; 0,6% Agarosegel; Sonde: pUC-MZ1.3, spez. Aktivität 3 x $10^8$ cpm/μg. Hybridisierungs-Puffer: 5 x SSC; 1 % SDS, 10 mM Tris/HCl (pH 8,0), 2 mM EDTA, 5 μg/ml t-RNA. Vorhybridisierung: 60°C, 3 Std.; Hybridisierung: 60°C, 20 Std., 1,6 x $10^6$ cpm/ml; Waschschritte: Temp.: 60°C; 1 x 30 min. 2 x SSC, 0,1% SDS; 2 x 30 min. 1 x SSC, 0,1 % SDS; 2 x 30 min., 0,5 x SSC, 0,1% SDS. Belichtung von Kodak XAR-5,3 Std. bei -70°C mit Verstärkerfolie.

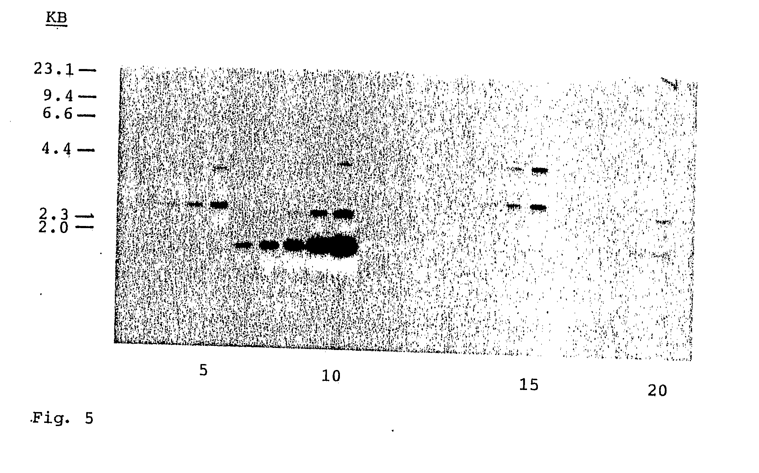

Figur 5: Vergleich von pUC-MZ1.3 und M13 Sonden im Southern Blot mit klonierten DNA-Fragmenten.
0,6 % Agarosegel; Spuren 1 - 5 und 11 - 15: Verdünnungsreihe von ClaI gespaltenem M13.mp8; Spuren 6 - 10 und 16-20: Verdünnungsreihe von PstI/BamHI gespaltenem pUC-MZ1.3 Jeweils 5 Verdünnungsschritte: 0,04 ng, 0,1 ng, 0,3 ng, 1 ng, 3 ng. Sonden: Spur 1 - 10 hybridisiert mit pUC-MZ1.3, spez. Aktiv.: $2 \times 10^9$ cpm/$\mu$g, $1 \times 10^6$ cpm/ml Hybr. Lsg. Spur 11 - 20 hybridisiert mit M13.mp8, spez. Aktivität: $2 \times 10^9$ cmp/$\mu$g, $1 \times 10^6$ cpm/ml Hybr. Lsg. Hybridisierungs-Puffer: $5 \times$ SSC; 1% SDS, 10 mM Tris/HCl (pH 8,0), 2mM EDTA, 5 $\mu$g/ml t-RNA, 2% Trockenmilchpulver. Vorhybridisierung: $60^\circ$ C, 3 Std.. Hybridisierung: $60^\circ$ C, 20 Std.. Waschschritte: $2 \times 15$ min. in $2 \times$ SSC, 0,1% SDS bei Raumtemp., $2 \times 30$ min. in $2 \times$ SSC, 0,1% SDS bei $65^\circ$ C. Belichtung von Kodak XAR-5 Röntgenfilm, 30 min. bei Raumtemperatur.

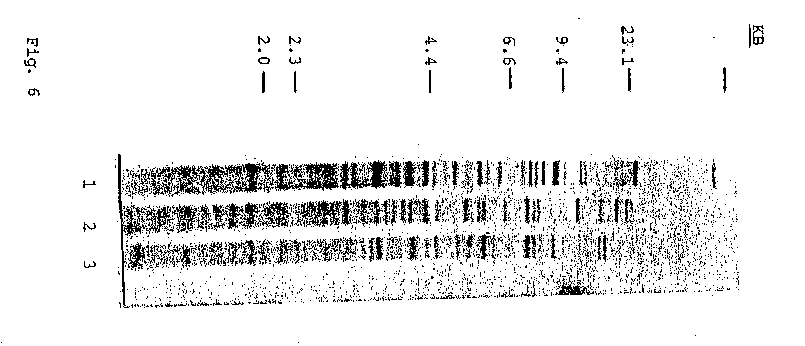

Figur 6: Analyse von RFLP-Fragmenten von drei nicht verwandten Blutspendern mit der enzymatisch markierten Sonde pUC-MZ1.3. Restriktionsspaltung: 10 $\mu$g DNA, 40 U Hinfl; 0,6% Agarosegel; Sonde: pUC-MZ1.3 markiert mit Biotin, Hybridisierungs-Puffer: $5 \times$ SSC; 1 % SDS, 10 mM Tris/HCl (pH 8,0), 2 mM EDTA, 5 $\mu$g/ml t-RNA. Vorhybridisierung: $60^\circ$ C, 3 Std. Hybridisierung: $60^\circ$ C, 20 Std., 1,6 $\times 10^6$ cpm/ml. Waschschritte: Temp.: $60^\circ$ C, $1 \times 30$ min., $2 \times$ SSC, 0,1% SDS; $2 \times 30$ min., $1 \times$ SSC, 0,1% SDS; $2 \times 30$ min., 0,5 $\times$ SSC, 0,1% SDS. Nachweis durch Streptavidin-Meerrettich-Peroxidase und TMB.

Die Beispiele erläutern die Erfindung

Material und Methoden

Die angewendeten Methoden sind, soweit nicht anders vermerkt, in "Molecular Cloning, A Laboratory Manual" von Maniatis, T., Fritsch E. F. und Sambrook, J., Cold Spring Harbor Laboratory, 1982 beschrieben.

Isolierung von menschlichen Sequenzen mit Homologie zu M13 Sequenzen

Genomische DNA aus menschlichen Lymphozyten wird mit einer Restriktionsendonuklease (vorzugsweise Sau3A) partiell gespalten. Die Fragmente werden in einer durch $T_4$-Ligase katalysierten Reaktion in ein für die Aufnahme der Fragmente vorbereitetes Lambda-Bakteriophagen-Genom (vorzugsweise das Bam HI gespaltene Genom des Phagen EMBL3) eingesetzt. Dann werden die Ligierungsprodukte mit Lambda Verpackungsextrakten (Packagene-Kit, Promega-Biotec, Madison, WI, USA) zu infektiösen Phagen zusammengesetzt. Bakterien eines für die Vermehrung der Phagen geeigneten Stammes (vorzugsweise E.coli NM539) werden mit mindestens $8 \times 10^5$ PFU (Plaque bildenden Einheiten) der Phagen infiziert und zur Amplifikation der Genbank ausplattiert. Die amplifizierten Phagen werden durch Elution von den Platten geerntet. Für die Herstellung der Genbank werden die bei Maniatis et al. (1982, S.256-307) beschriebenen Methoden eingesetzt. Eine detaillierte Darstellung der Herstellung der Genbank ist bei Schneider, P.M. (Dissertation, Mainz 1987) zu finden.

Die Identifizierung von Lambda-Phagen, deren Insertionen Homologie zur repetitiven Sequenz im Protein III Gen des Bakteriophagen M13 aufweisen, erfolgt nach der in situ Hybridisierungs-Methode von Benton und Davis (Science 196, 180 - 182, 1977): Bakterien eines geeigneten Stammes (vorzugsweise E. coli Q358) werden mit einem Aliquot der amplifizierten Phagenbank ($10^5$ - $10^6$ PFU) infiziert und auf Agar Platten mit einer Dichte von 150 - 300 Plaques/cm$^2$ ausplattiert. Von den Platten werden Abzüge auf Nitrozellulosefiltern (BA 85, Schleicher und Schüll, Dassel; Biotrace NT, Gelman Sciences, Ann Arbor, MI, USA) erstellt. Nach Freisetzung, Denaturieren und Fixieren der Phagen-DNA werden die Filter mit einer Sonde, die das vollständige Genom des Bakteriophagen M13mp8 umfaßt, oder einer Sonde, die das NciI/ClaI-Fragment aus dem Protein III des Bakteriophagen M13 enthält, hybridisiert. Die Sonde wird vor der Hybridisierung mit dem radioaktiven Phosphorisotop $^{32}$P durch Nicktranslation markiert. Die Vorhybridisierung der Filter erfolgt in $6 \times$ SSC (hergestellt aus einer Stammlösung von $20 \times$ SSC: 3M NaCl, 0,3M Natrium Citrat, pH 7,0), 40% Formamid, 5mM EDTA (Äthylendiamintetraacetat) und 0,25% Trockenmilchpulver bei $42^\circ$ C für 4 Stunden. Rom cm$^2$ Filterfläche werden 0,15 ml des Puffers eingesetzt. Die Hybridisierung findet im gleichen Puffer unter Zusatz der markierten Sonde ($1 \times 10^6$ cpm/ml) statt. Die Hybridisierungszeit beträgt 15 Std., pro cm$^2$ Filterfläche werden 0,15 ml des Puffers eingesetzt. Nach der Hybridisierung werden die Filter wie folgt gewaschen: $2 \times 15$ min. in $2 \times$ SSC, 0,1% SDS (Natrium Dodecylsulfat) bei Raumtemperatur, $2 \times 15$ min. in 2xSSC, 0,1% SDS bei $50^\circ$ C, $2 \times 15$ min. in 2xSSC, 0,1% SDS bei $65^\circ$ C, $1 \times 15$ min. in $1 \times$ SSC, 0,1% SDS bei $65^\circ$ C, $1 \times 15$ min. in $1 \times$ SSC ohne SDS bei $65^\circ$ C. Die Filter werden nach dem Trocknen an der Luft bei Raumtemperatur 5 Tage auf Röntgenfilm (Fuji RX NEW) bei -70$^\circ$ C mit Verstärkerfolie exponiert. Lambda Plaques, welche mit der Sonde positive Signale geben, werden aus den Genbank-Platten ausgestochen und Phagen dieser Plaques werden in geringer Konzentration erneut plattiert. Einzelplaques, welche mit der M13-Sonde reagieren, werden durch eine erneute Hybridisierung wie oben beschrieben identifiziert. Von den nach dieser Hybridisierung verbleiben positiven Lambda-Klonen wird DNA isoliert. Die Methoden der Identifizierung und Isolierung sind im Detail bei Maniatis et al. (1982, S.309-373) beschrieben.

## Isolierung von DNA aus Lambda Bakteriophagen

Bakteriophagen aus Einzelplaques werden in 4 ml-Kulturen in geeigneten Wirtsstämmen (vorzugsweise E.coli Q358) vermehrt (Maniatis et al., 1982, S.373). Kleine DNA-Mengen werden aus diesen Kulturen nach der bei Maniatis et al. (1982, S. 371) beschriebenen Methode isoliert. Für Präparationen größerer Mengen von Lambda-DNA werden 5 x $10^8$ Phagen aus der 4ml-Kultur mit $10^{10}$ Bakterien (E.coli Q358) gemischt und in 500 ml-Kulturen weiter expandiert (Maniatis et al., 1982, S.77-78). Die Isolierung der Phagen aus der Kultur, Reinigung über CsCl-Gradienten und die Extraktion der DNA wird nach Maniatis et al. (1982, S.80-83) durchgeführt.

## Isolierung von Plasmid DNA

Plasmid-DNA aus Kleinkulturen von 2 - 3 ml wird nach der bei Maniatis et al. (1982, S.368-369) beschriebenen Methode der alkalischen Lyse isoliert. Größere Mengen von Plasmid DNA werden nach der Methode von Garger et al. (Biochem. Biophys. Res. Comm. 117, 835, 1983) aus Kulturen von 1 oder 2 l präpariert.

## Isolierung von doppelsträngiger DNA des Bakteriophagen M13mp8

Die Reinigung von doppelsträngiger M13mp8 DNA (replikative Form) wird nach der Methode von Messing, J. (Meth. Enzymol. 101, 20-79, 1983) durchgeführt.

## Restriktionskartierung von Plasmid- und Bakteriophagen-DNA

Die aus Bakterien- oder Phagenkulturen isolierte DNA wird mit Restriktionsenzymen gespalten (Maniatis et al., 1982, S.98-106), die entstandenen Fragmente werden elektrophoretisch auf Agarosegelen aufgetrennt und mit Ethidium-Bromid nachgewiesen (Maniatis et al., 1982, S.150-163). Als DNA-Längen-Standards dienen mit dem Restriktionsenzym HindIII gespaltene DNA des Bakteriophagen Lambda (Gibco BRL, Eggenstein) und mit dem Restriktionsenzym HaeIII gespaltene DNA des Bakteriophagen PhiX174 (New England Biolabs, Schwalbach). Restriktionskarten werden nach den Ergebnissen von partiellen Spaltungen und Spaltungen mit mehreren Restriktionsenzymen erstellt.

## Isolierung von Restriktionsfragmenten und Subklonierung von Fragmenten in Plasmiden

Restriktionsfragmente von Plasmiden oder Bakteriophagen DNA werden durch präparative Gelelektrophorese (Dretzen, G. et al., Anal. Biochem. 12, 295-298, 1981) isoliert. Für die Ligation von isolierten Fragmente wird $T_4$-Ligase (Pharmacia LKB GmbH, Freiburg) benutzt. Für die Transformation der Plasmide (vorzugsweise pUC-18) in geeignete Bakterienstämme (Yanisch-Perron, C. et al., Gene 33, 103 - 119, 1985) wird die Method von Hahahan, D., (J.Mol.Biol.166, 557 - 580, 1983) verwendet.

## Radioaktive Markierung von DNA-Sonden

Zur Herstellung radioaktiv markierter Sonden wird gereinigte Plasmid- oder Bakteriophagen-DNA entweder durch Nicktranslation (Nick Translation Reagent Kit, Gibco BRL, Eggenstein) oder Random Priming Reaktionen (Random Primed DNA Labeling Kit, Boehringer Mannheim GmbH, Mannheim; Multiprime DNA labelling system, Amersham Buchler, Braunschweig) nach Angaben der Kit-Hersteller mit radioaktiven desoxy-Adenosin-Triphosphaten markiert. Für die Markierung werden radioaktive Nukleosid-Triphosphate (Alpha $^{32}$PdATP; DuPont, NEN Product Division, Dreieich; Amersham Buchler, Braunschweig) mit einer spezifischen Aktivität von 3000Ci/mmol eingesetzt.

## Biotinylierung von DNA Sonden

Bioten-markierte Sonden werden durch Nicktranslation (Nick Translation Reagent Kit, Gibco BRL, Eggenstein) mit biotinyliertem desoxy-Uridin-Triphosphat (Biotin-11 - dUTP, Gibco BRL, Eggenstein; Enzo Biochem Inc., New York, NY, USA) nach Angaben des Kit-Herstellers hergestellt.

## Southern Blot-Analysen von clonierter DNA

Restriktionsfragmente von Plasmid- oder Bakteriophagen-DNA werden wie im Abschnitt Restriktionskartierung beschrieben erhalten, elektrophoretisch aufgetrennt und nachgewiesen. Anschließend werden die Fragmente auf Nitrozellulose-Membranen (BA85), Schleicher und Schuell; Biotrace NT, Gelman Sciences) oder Nylon-Membranen (Biotrace RP, Gelman Sciences) übertragen. Die Methode des Southern Blots ist von Southern, E. (J. Mol. Biol. 98, 503-517, 1975) entwickelt worden und bei Maniatis et al. (1982, S.374-389) ausführlich beschrieben. Für die Übertragung von DNA auf Nylon-Filter wird das alkalische Transfer Protokoll von Reed und Mann (Nucl. Acids Res. 13, 7207-7221, 1985) benutzt. Nach dem Transfer werden die Filter 2 Stunden bei 80°C gebacken.

Der Hybridisierungpuffer besteht aus 5xSSC, 1% SDS, 10mM Tris (Tris-hydroxymethyl-aminomethan, mit HCl auf pH 8,0 eingestellt), 2 mM EDTA pH 8,0 5 μg/ml t-RNA aud Hefe (Boehringer Mannheim GmbH, Mannheim) und 2% Magermilchpulver. Für die Vorhybridisierung werden die Filter in diesem Puffer für 3 Stunden bei 60°C ohne die markierte Sonde inkubiert. Danach folgen 20 Stunden Hybridisierung im gleichen Puffer unter Zusatz der markierten Sonde ebenfalls bei 60°C. Nach Abschluß der Hybridisierung werden die Filter einer Reihe von Waschschritten mit absteigenden Konzentrationen von SSC und einer konstanten Konzentration von 0,1 % SDS bei 65°C unterzogen. Nach dem Trocknen bei Raumtemperatur werden die Filter auf Kodak XAR-5 Röntgenfilm bei -70°C mit Verstärkerfolie exponiert.

## Southern Blot-Analysen mit menschlicher DNA

Die für den Southern Blot mit menschlicher DNA verwendeten Methoden sind weitgehend identisch mit denen, die oben für Southern Blots mit klonierter DNA beschrieben werden. Abweichungen sind in den folgenden Abschnitten angegeben.

Pro Analyse werden 10μg menschliche DNA mit dem Restriktionsenzym HinfI vollständig gespalten. Die Restriktionsfragmente werden auf einem 0,6% Agarosegel getrennt. Spaltung und Trennung der Fragmente wird nach Färbung der Gele mit Ethidium Bromid kontrolliert, als DNA-Längenstandard dient mit dem Restriktionsenzym HindIII gespaltene DNA des Bakteriophagen Lambda (Gibco BRL, Eggenstein).

Die Übertragung der DNA auf Nylonfilter (Gelman Sciences) erfolgt durch alkalischen Transfer (Reed, K.C. und Mann, D.A., Nucl. Acids Res. 13, 7207-7221, 1985).

Die Filter werden entweder mit radioaktiv markierten Sonden oder mit biotinylierten Sonden inkubiert. Der Puffer für die Vorhybridisierung besteht aus 5xSSC, 1% SDS, 10 mM Tris/HCl pH 8,0, 2 mM EDTA pH 8,0, 5 μg/ml t-RNA aus Hefe (Boehringer Mannheim GmbH, Mannheim). Zur Reduzierung des Hintergrundes können außerdem 2% Magermilchpulver zugesetzt werden. Die Hybridisierung findet in der gleichen Pufferlösung unter Zusatz der Sonde statt. Die Hybridisierungen werden bei 60°C durchgeführt. Die Vorhybridisierungszeit beträgt 2 - 3 Stunden, die Hybridisierungszeit 20 Stunden. Nach der Hybridisierung werden die Filter einer Serie von Stringenz-Waschschritten unterworfen. Dazu werden die Filter in Puffer-Lösungen absteigender Salzkonzentration (SSC) mit Zusatz von 0,1% SDS bei 60°C inkubiert. Filter, die mit radioaktiv markierten Sonden inkubiert wurden, werden anschließend bei Raumtemperatur getrocknet und bei -70°C mit Verstärkerfolie auf Röntgenfilm (Kodak XAR-5) exponiert. Bei Filtern, die mit Biotinmarkierten Sonden inkubiert wurden, folgen nach dem Waschen die für den enzymatischen Nachweis beschriebenen Inkubationsschritte.

## Nicht-radioaktiver Nachweis von RFLP-Fragmenten auf Southern Blot-Filtern

Für den enzymatischen Nachweis von RFLP-Fragmenten wird ein Streptavidin-Meerrettich-Peroxidase-Komplex (See-Quence™ HLA-D Kit Cetus Corporation, Emeryville, CA., USA) oder ein Avidin-Meerrettich-Peroxidase-Komplex (Vectastain™ ABC ELITE Kit, Vector Laboratories, Burlingame, Ca., USA) mit dem Meerrettich-Peroxidase-Substrat 3,3',5,5'-Tetramethylbenzidin (TMB, Sigma, München) verwendet.

Das Arbeitsprotokoll folgt bis auf einige Modifikationen dem von Sheldon et al. (Proc. Natl. Acad. Sci. USA 83, 9085 - 9089, 1986) beschrieben:

Nach dem letzten Stringenz-Waschschritt werden die Filter 5 min. in einer Puffer-Lösung (A) aus 5% (Vol./Vol.) Triton X-100, 2,7mM KCL, 237mM NaCl, 1,5mM $KH_2PO_4$ und 8mM $Na_2HPO_4$ mit einem pH-Wert von 7,4 gewaschen. Anschließend werden die Filter in dem gleichen Puffer unter Zusatz des nach Angaben des Herstellers verdünnten Streptavidin/Meerettich-Peroxidas-Komplexes 40 min. inkubiert. Danch folgen 5 Waschschritte von je 5 min. in Puffer-Lösung (B), welche aus Puffer-Lösung (A) mit einem Zusatz von 1M Harnstoff und 1% Dextransulfat (Mol. Gew. 500000) besteht. Die Filter werden dann einmal 5 min. in einer Puffer-Lösung von 0,01M Natrium Citrat und 0,01M EDTA (pH 5,0) gewaschen. Anschließend werden sie in der gleichen Puffer-Lösung unter Zusatz von TMB 5 min. inkubiert. Das TMB wird zurvor in 100% Ethanol gelöst (Konzentration: 2mg/ml). Für die Inkubation wird eine Mischung aus 1 Teil ethanolischer TMB-Stammlösung mit 19 Teilen des Citrat/EDTA Puffers benutzt. Zur Farbentwicklung werden die Filter in eine Puffer-Lösung von pH 5,0 überführt, die wie die vorige aus 0,01M Natrium Citrat, 0,01M EDTA, 0,1mg/ml TMB und 5% Ethanol besteht, der aber unmittelbar vor der Benutzung 0,0014% $H_2O_2$ zugesetzt wurden. In dieser Lösung werden die Filter 30 bis 60 min. inkubiert. Die Farbentwicklung wird durch 4-maliges Waschen in destilliertem Wasser gestoppt. Die Filter werden in feuchtem Zustand in Plastikfolie eingeschweißt und photographiert. Alle Inkubationen werden bei Raumtemperatur durchgeführt.

## DNA-Sequenzierung

Die DNA-Sequenzierung wird nach der Methode von Chen, E.Y., und Seeburg, P.H. (DNA 4, 165, 1985) mit einem pUC-Sequenzierungs-Kit (Boehringer Mannheim GmbH, Mannheim) nach der Vorschrift des Herstellers durchgeführt. Zur Markierung wird $^{35}$S-dATP (spez. Aktivität über 1000Ci/mol; Amersham Buchler, Braunschweig) eingesetzt. Für die Gelelektrophorese werden 6 - 8 %ige Polyacrylamid Gele verwendet. Mit den Gelen wird ein Röntgenfilm (Fuji RX NEW) 2 Tage belichtet.

## DNA-Synthese

DNA-Fragmente werden auf einem DNA-Synthesizer 381 A (Applied Biosystems, Darmstadt-Eberstadt) nach der $\beta$-Cyanäthylphosphoramidit-Methode nach der Vorschrift des Geräteherstellers synthetisiert. Zur Reinigung der Fragmente wird eine DEAE-Ionenaustauscher-HPLC-Säule benutzt.

## Beispiel 1:

### Isolierung der Sonde und Hybridisierungsexperimente

### Isolierung der Sonde

Das Genom des Bakteriophagen M13 enthält repetitive Sequenzen, welche mit Minisatelliten im menschlichen Genom hybridisieren (Vassart, G., et al., Science 235, 683 - 684, 1987). DNA aus M13 kann somit als Sonde zur Identifizierung solcher Minisatelliten eingesetzt werden. Fragmente des menschlichen Genoms, die Sequenzen mit Homologie zur M13-Sonde enthalten, lassen sich aus einer menschlichen Genbank in Lambda-Bakteriophagen isolieren und durch Southern Blot-Analyse der isolierten Phagen-Klone charakterisieren. Ein Beispiel für ein solches Fragment aus dem menschlichen Genom ist die Insertion des Lambda-Klons MZ1.3, die ein 1,9 kb langes PstI/BamHI-Fragment mit Homologie zum M13-Genom enthält (Fig. 1).

### Subklonierung und Restriktionskartierung der Sonde

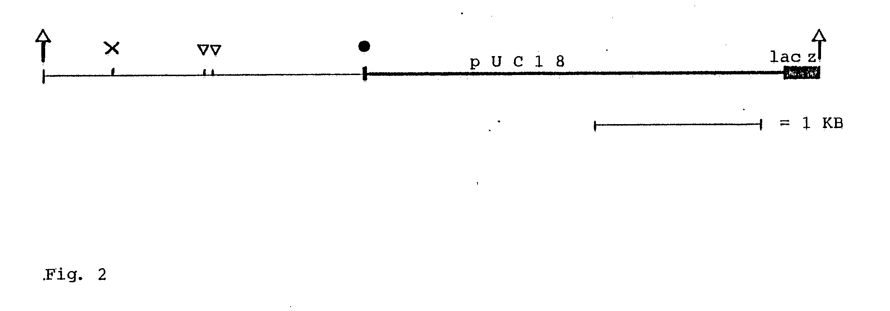

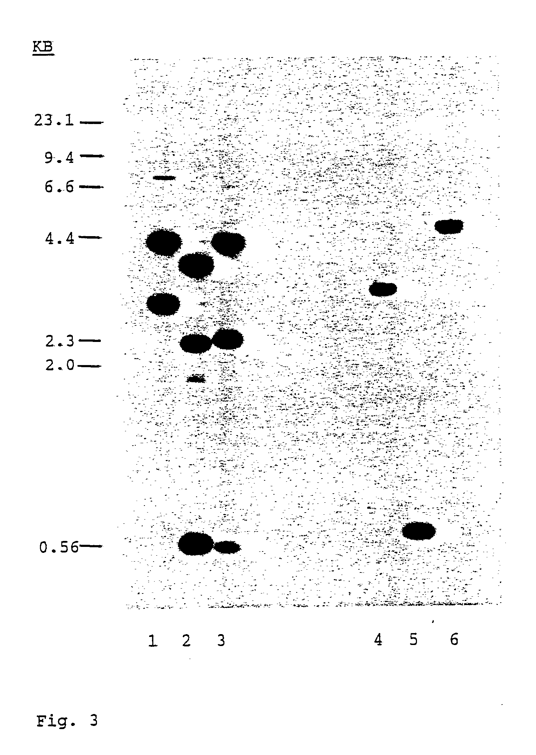

Für die Verwendung als Sonde ist es zweckmäßig, die in Lambda-Phagen identifizierten Fragmente in Bakterienplasmide zu übertragen. Dazu wird das Fragment gelelektrophoretisch gereinigt, isoliert und in ein als Vektor geeignetes Plasmid eingesetzt. Für die Aufnahme von PstI/BamHI-Fragmenten sind die Vektoren der pUC Reihe (Yanisch-Perron C., et al., Gene 33, 103 - 119, 1985) geeignet. Das Plasmid pUC-MZ1.3 besteht aus dem Vektor pUC-18 und dem 1,9 kb langen PstI/BamHI-Fragment des Lambda-Klons MZ1.3, welches über die entsprechenden Spaltstellen in den Vektor eingesetzt ist. Eine Restriktionsanalyse des Plasmids pUC-MZ1.3 ergibt die in Fig. 2 gezeigt Karte. Durch Southern Blot-Analysen kann der zur Insertion des Plasmids pUC-MZ1.3 homologe Bereich im M13-Genom auf ein 0,6 KB langes NciI/ClaI-Fragment, das die erwähnte repetitive Sequenz enthält, eingeschränkt werden (Fig. 3). Die Restriktionsanalyse der pUC-MZ1.3 Insertion im Southern Blot zeigt, daß 4 Fragmente der Insertion, welche durch gleichzeitige Spaltung mit den Restriktionsenzymen Sau 3A und MspI zu erhalten sind mit einem M13-Fragment, welches die repetitive Sequenz enthält, hybridisieren (Fig. 1). Die 1,9 kb Banden in den Spuren 3 und 5, die 1 kb Bande in Spur 3 und die 0,9 kb Bande in Spur 4 sind auf partielle Spaltung zurückzuführen.

### Vergleich der Sonden pUC-MZ1.3 und M13mp8 in Hybridisierungsexperimenten

Das Plasmid pUC-MZ1.3 eignet sich als Sonde zum Nachweis von RFLPs in menschlichen Genomen mit der Southern Blot-Technik. Zum radioaktiven Nachweis der Polymorphismen, für den die Sonde durch den Einbau von $^{32}$P-enthaltenden Nukleotiden markiert wird, reichen sehr kurze Expositionszeiten (Fig. 4). Die hohe Nachweisempfindlichkeit der Sonde ergibt sich aus der hohen Zahl von Sondenmolekülen, die von einer Zielsequenz mit Homologie zur Insertion des Plasmids gebunden werden. Die im Vergleich zu anderen Sonden-Sequenzen mit vollständiger Homologie zu einer Zielsequenz erhöhte Bindung der pUC-MZ1.3-Insertion an eine vollständig homologe Zielsequenz ist in Hybridisierungsexperimenten nachweisbar, in denen Restriktions-Fragmente von pUC-MZ1.3 und M13mp8 mit dem vollständigen Plamid pUC-MZ1.3 oder dem vollständigen M13mp8-Genom als Sonde untersucht werden (Fig. 5). Die Insertion des Plasmids pUC-MZ1.3 (1,9 kb Bande) bindet etwa 10fach mehr Moleküle der Sonde pUC-MZ1.3 als der Vektoranteil (2,7 KB Bande), obwohl die Sonde vollständige Homologie zu beiden Fragmenten besitzt (Fig. 5, , Spur 6 - 10). Eine Messung der Radioaktivität in den 3 Banden von Spur 10 nach Cerenkov ergab 3,7 x $10^3$ cpm für die 2,7 kb Bande, 3,5 x $10^4$ cpm für die 1,9 kb Bande. Die 4,7 kb Bande, für die 5 x $10^2$ cpm gemessen wurden, ist auf unvollständige Spaltung zurückzuführen und entspricht dem linearisierten, vollständigen pUC-MZ1.3 Plasmid. Wird M13mp8 DNA als Sonde zum Nachweis von M13mp8 Fragmenten benutzt, so binden das 2,9 kb Fragment, welches die zur MZ1.3-Insertion homologe repetitive Sequenz trägt, und das 4,4 kb Restfragment des Phagen etwa gleich viele Sondenmoleküle (Fig. 5, Spur 11 - 15). Im Gegensatz zu der Insertion von pUC-MZ1.3 zeigt also die zu der Insertion homologe Region im M13-Genom keine verstärkte Bindung von Sondenmolekülen. Homologien zwischen M13 und pUC-MZ1.3 sind aus Fig. 5 (Spur 1 - 5) und Fig. 5 (Spur 16 - 20) erkennbar. Die 1,9 kb Bande des Plasmids und die 2,6 kb Bande von M13 sind durch Hybridisierung zwischen der Insertion des Plasmids und dem dazu homologen M13-Fragment zu erklären. Die 2,7 kb Bande des Plasmids und die 4,4 kb Bande des Phagen weisen eine Homologie durch die ihnen beiden gemeinsamen Sequenzen des lac-Operons auf (Vieira, J. und Messing, J., Gene 19, 259 - 268, 1982).

Die Ergebnisse zeigen daß der für die Insertion von pUC-MZ1.3 gefundene Verstärkungseffekt sowohl von der Sequenz des Sonden moleküls als auch des Zielmoleküls abhängig ist. Der limitierende Schritt für die Verstärkung ist somit die Reaktion zwischen dem Zielmolekül und dem ersten daran gebundenen Sondenmolekül. Wird das erste Sondenmolekül über eine nicht-verstärkende Sequenz, in Fig. 5 die Vektor Sequenz, an das Ziel-Molekül gebunden, tritt keine Verstärkung auf.

Die Voraussetzungen, welche als Bedingung für die Signalverstärkung an das Zielmolekül gestellt werden, werden nicht nur von der Insertion des Plasmids pUC-MZ1.3 erfüllt, sondern auch von einer Vielzahl von Sequenzen im menschlichen Genom, welche durch die Sonde nachgewiesen werden. Diese Aussage wird belegt durch die oben erwähnte kurze Belichtungszeit für Southern Blots nach Hybridisierung mit der radioaktiv markierten Sonde pUC-MZ1.3 (Fig.4). Es ist deshalb davon auszugehen, daß neben dem erfindungsgemäßen Fragment MZ1.3 auch andere mit M13 hybridisierende Fragmente des menschlichen Genoms ähnliche Verstärkungseffekte zeigen, wenn sie als Sonden eingesetzt werden. Weiterhin ist davon auszugehen,daß auch aus den Genomen anderer Eukaryonten, die mit M13 nachweisbare Minisatelliten enthalten, solche Fragmente isoliert werden können.

Der mit der Sonde pUC-MZ1.3 gefundene Verstärkungseffekt in der Bindung an das Zielmolekül erlaubt den Einsatz von nichtradioaktiven Nachweismethoden, die eine geringere Empfindlichkeit als der radioaktive Nachweis mit $^{32}$P haben. Figure 6 zeigt das Ergebnis eines Southern Blot-Experimentes mit einer mit Biotin markierten pUC-MZ1.3 - Sonde und enzymatischem Nachweis. In Experimenten, in denen unter gleichen Bedingungen biotinylierte M13-DNA als Sonde eingesetzt wurde, sind keine Banden nachweisbar.

Beispiel 2:

Bestimmung der in pUC-MZ1.3 enthaltenen, zum Nachweis von menschlichen Minisatelliten geeigneten Nukleinsäure-Sequenzen

Die DNA-Sequenz des in pUC-MZ1.3 enthaltenen menschlichen Nukleinsäure-Fragments wird nach Chen, E.Y. und Seeburg, P.H. (DNA 4, 165, 1985) bestimmt.

Das Plasmid pUC-MZ1.3 enthält repetitive Sequenzen deren Grundmotiv die folgende Basensequenz aufweist:
5′- CGGCGGWGGTGGTGGTATYGGTTGTGS - 3′
in der W entweder T oder A , S entweder C oder G, und Y entweder C oder T bedeutet.

Das 27 Basenpaare lange Grundmotiv enthält eine zum Nachweis von Minisatelliten, die mit der Sonde pUC-MZ1.3 hybridisieren, notwendige Sequenz: Banden, die im Southern Blot mit der Sonde pUC-MZ1.3 nachweisbar sind (Figur 4), werden auch durch die Sonde pUC-MZR7 nachgewiesen. Letztere besteht aus einem im Vektor pUC18 klonierten Fragment, welches 7 Wiederholungen des 27 Basenpaare langen Grundmotivs enthält.

Die ermittelte Sequenz gestattet die Synthese erfindungsgemäßer Nukleinsäure-Fragmente.

## Ansprüche

1. Nukleinsäure-Fragment aus einem eukaryontischen Genom, das die folgenden Merkmale aufweist:
(a) es ist erhältlich durch Absuchen einer Genbank eines Eukaryonten mit dem Ncil/Clal-Fragment aus dem Protein III-Gen des Bakteriophagen M13; und
(b) es verursacht die Bindung von mindestens fünfmal mehr rekombinanten DNA-Molekülen, in denen es enthalten ist, an eine zu ihm homologe Zielsequenz als eine Vektor-DNA an eine zu ihr homologe Zielsequenz.

2. Nukleinsäure-Fragment nach Anspruch 1, das eine mindestens einmal widerholte Sequenz mit der Basenreihenfolge
5′- CGGCGGWGGTGGTGGTATYGGTTGTGS - 3′

enthält, in der W entweder T oder A, S entweder G oder C, und Y entweder C oder T bedeutet.

3. Nukleinsäure-Fragment nach Anspruch 1 oder 2, das aus dem Genom eines Säugers stammt.

4. Nukleinsäure-Fragment nach einem der Ansprüche 1 bis 3, das aus dem Genom eines Menschen stammt.

5. Nukleinsäure-Fragment nach Anspruch 4, das in dem rekombinanten Plasmid pUC-MZ1.3, DSM 4772 enthalten ist.

6. Nukleinsäure-Fragment nach einem der Ansprüche 1 bis 5, das durch DNA-Synthese hergestellt wurde.

7. Nukleinsäure-Fragment nach einem der Ansprüche 1 bis 6, das als Doppelstrang vorliegt.

8. Nukleinsäure-Fragment nach einem der Ansprüche 1 bis 7, das aus zwei partiell bis vollständig homologen Einzelsträngen besteht.

9. Nukleinsäure-Fragment nach einem der Ansprüche 1 bis 8, das mit einem Isotop markiert ist.

10. Nukleinsäure-Fragment nach einem der Ansprüche 1 bis 9, das mit einer fluoreszierenden oder enzymatischen Markierung oder mit einer chemischen Gruppe versehen ist, die als molekulare Brücke zu einer derartigen Markierung dient.

11. Verfahren zur Isolierung eines Nukleinsäure-Fragments nach einem der Ansprüche 1 bis 8, bei dem man das Nukleinsäure-Fragment in einer eukaryontischen Genbank durch Hybridisierung mit einem vollständigen M13-Genom, vorzugsweise mit dem NciI/ClaI-Fragment aus dem Protein III-Gen des M13-Genoms, identifiziert und sodann aus der Genbank isoliert.

12. Verfahren zur Isolierung eines Nukleinsäure-Fragments nach einem der Ansprüche 1 bis 8, bei dem man das Nukleinsäure-Fragment in einer eukaryontischen Genbank durch Hybridisierung mit einem nach Anspruch 11 erhaltenen Nukleinsäure-Fragment identifiziert und sodann aus der Genbank isoliert.

13. Verwendung eines Nukleinsäure-Fragments nach einem der Ansprüche 1 bis 10 zum Nachweis von Restriktions-Fragment-Längen-Polymorphismen (RFLPs) in eukaryontischen Genomen.

14. Verwendung nach Anspruch 13, wobei das eukaryontische Genom das Genom eines Säugers ist.

15. Verwendung nach Anspruch 14, wobei das eukaryontische Genom das Genom eines Menschen ist.

Fig. 1 a

Fig. 1b

Fig. 2

pUC18

lac z

⊢————⊢ = 1 KB

EP 0 357 028 A2

Fig. 3

Fig . 4

Fig. 5

Fig. 6